# EUROPEAN PATENT APPLICATION

(11) **EP 2 462 960 A2**
(43) Date of publication of application: **13.06.2012**
(21) Application number: 11192717.4
(22) Date of filing: 09.12.2011
(51) Int. Cl.: A61L 29/06, A61L 29/16

(54) **Polymer for controlling delivery of bioactive agents and method of use**

(30) Priority: 09.12.2010 US 964316
(71) Applicant: Teleflex Medical Incorporated, Durham, NC 27709 (US)
(72) Inventor: Do, Hiep, Sinking Spring, PA Pennsylvania 19608 (US); Rosenblatt, Joel, Pottstown, PA Pennsylvania 19465 (US)
(74) Representative: von Hirschhausen, Helge

(57) **Abstract**

The invention claims a device having an antimicrobial agent, the device comprising a base material; and a chlorhexidine or pharmaceutically acceptable salt thereof disposed in the base material sufficient to reduce microbial growth, the base material including a polymer having a silicone monomer and a urethane monomer.

## Description

### FIELD OF THE INVENTION

The present invention generally relates to a polymer for controlling delivery of a bioactive agent. More particularly, the present invention pertains to a polymer for controlling delivery of a bioactive agent and a method of use in an implantable medical device.

### BACKGROUND OF THE INVENTION

Catheters are presently utilized in a great variety of medical procedures where they provide a great benefit to patients and medical practitioners. Unfortunately, conventional catheters are capable of being contaminated with microorganisms. Catheter-related infections are thought to arise by several different mechanisms. Contamination of the point of entry into the patient and subsequent colonization of catheters by microbes as well as formation of a bacterial biofilm on the external and internal surfaces are thought to be the major routes for catheter related blood stream infections (CRBSI). To address this problem, catheters may include an antimicrobial agent. Unfortunately, conventional polymers are unsuitable for use in long term indwelling medical devices.

Accordingly, it is desirable to provide a polymer suitable for controlling the release of a bioactive agent from an indwelling medical device that is capable of overcoming the disadvantages described herein at least to some extent.

### SUMMARY OF THE INVENTION

The foregoing needs are met, to a great extent, by the present invention, wherein in some respects a polymer suitable for controlling the release of a bioactive agent from an indwelling medical device and a catheter having such a polymer and bioactive agent is provided.

An embodiment of the present invention pertains to a medical device having an antimicrobial agent. The medical device includes a base material and chlorhexidine or a pharmaceutically acceptable salt thereof disposed in the base material sufficient to reduce microbial growth. The base material includes a polymer having a silicone monomer and a urethane monomer.

Another embodiment of the present invention relates to a medical catheter including an elongated hollow tube, an exterior surface of the elongated hollow tube having a base material, and chlorhexidine or a pharmaceutically acceptable salt thereof disposed in the base material sufficient to reduce microbial growth. The base material having a polymer having a silicone monomer and a urethane monomer.

In a particular embodiment of the invention, the base material has a silicone:urethane ratio (wt/wt) of about 50:50 to about 80:20. In another particular embodiment of the invention, the base material has a silicone:urethane ratio (wt/wt) of about 65:35 to about 70:30.

There has thus been outlined, rather broadly, certain embodiments of the invention in order that the detailed description thereof herein may be better understood, and in order that the present contribution to the art may be better appreciated. There are, of course, additional embodiments of the invention that will be described below and which will form the subject matter of the claims appended hereto.

In this respect, before explaining at least one embodiment of the invention in detail, it is to be understood that the invention is not limited in its application to the details of construction and to the arrangements of the component set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present invention. It is important, therefore, that the claims be regarded as including such equivalent constructions insofar as they do not depart from the spirit and scope of the present invention.

### BRIEF DESCRIPTION OF THE DRAWINGS

FIG. 1 is a graph of cumulative amount of chlorhexidine released versus day for different copolymer of silicone-urethane according to embodiments of the invention on suitable silicone urinary catheters.

FIG 2 is a graph of cumulative percent chlorhexidine release versus day for different copolymer of silicone-urethane according to embodiments of the invention on suitable silicone urinary catheters.

### DETAILED DESCRIPTION

Embodiments of the invention provide a polymer for controlling delivery of a bioactive agent from an implantable medical device, an implantable medical device having the polymer, and a method of controlling delivery of the bioactive agent from the implantable medical device with the polymer by modulating the hydrophilicity vs. hydrophobicity. In various embodiments, the implantable medical device is a catheter, medical tubing, and/or other such medical devices that would benefit from having a broad spectrum of antimicrobial and/or antifungal activity over a prolonged period such as devices that interface with blood, blood products, and/or fibrinogenic fluids, tissues, and/or products. In addition, the bioactive agent may include any suitable agent for use in an implantable medical device. In general, examples of suitable bioactive agents include antibiotics, antimicrobials, antiseptics, antithrombogenics, fibrinolytics, anticoagulants (particularly heparin), anti-inflammatory agents, anti-pain agents, vasodilators, antiproliferatives, antifibrotics, growth factors, cytokines, antibodies, peptides and peptide mimetics, nucleic acids, and/or the like either alone or in combination with other agents. More particular examples of suitable bioactive agents include chlorhexidine and suitable pharmacological variations and salts thereof. In a specific example, the bioactive agent includes chlorhexidine diacetate (CHA) at a sufficient concentration to reduce or prevent microbial growth in and around the catheter.

Hydrophilic silicone-urethane coatings with high moisture content release chlorhexidine diacetate rapidly (greater than 80% of content released within a few days). Hydrophobic silicone coatings with low moisture content release very little of their content (less than 10%) within a few days. In contrast, improved silicone-urethane copolymers such as those described herein can progressively release chlorhexidine diacetate for longer than 14 days. This type of release profile is important for long indwelling medical devices that can pose an infection risk.

We have surprisingly found that it is possible to coat chlorhexidine onto a hydrophobic silicone substrate using a hydrophilic copolymer of silicone-urethane. A preferred hydrophilic copolymer of silicone-urethane has a moisture content greater than 0.6% but less than 1.8% and is more preferably between 0.7 and 1.0 %. The silicone monomer makes up the majority of the copolymer of silicone-urethane. For example, the copolymer of silicone-urethane may include about 50% to about 90% silicone. It is to be noted that the term "polymer", "silicone-urethane", "Si/Pu", "silicono-urethane copolymer" and variations thereof are used throughout interchangeably. In this regard, it is the ratio of silicone to urethane that determines the hydrophilicity vs. hydrophobicity of the resulting polymer. The urethane monomer content may range from about 10% to about 50%. In a particular example, the polymer is a Grade E5325 polymer which contains about 98 wt% polydimethylsiloxane (PDMS) and about 2% polyhexamethylenoxide (PHMO). Other examples of suitable polymers are manufactured by AorTech Biomaterials of Salt Lake City, UT, U.S.A. More particular examples of suitable polymers are described in U.S. Patent No. 6,313,254 entitled, POLYSILOXANE-CONTAINING POLYURETHANE ELASTOMERIC COMPOSITIONS, the disclosure of which is incorporated in its entirety. Of note, while polymers having silicone and urethane monomers are described herein, the polymer may include carbonate monomers to increase biostability. For example, the polymer may include about 1% to about 40% carbonate monomer.

Experiments performed are described hereinbelow which incorporate chlorhexidine diacetate (CHA) into different copolymers of silicone-urethane by spraying coating technique. In these experiments, copolymers of silicone-urethane were dissolved in 70/30 w/w dimethylforamide/tetrahydrofuran and then chlorhexidine diacetate was added to the polymer solution. The coating was applied by spray coating using a suitable low pressure nozzle. Examples of suitable low pressure nozzles include those manufactured by Nordson EFD Corporation of Westlake, OH 44145 USA.

### METHODS

### Example 1: Coating with 14.5% silicone-urethane copolymer / 4% CHA solution

14.5g of silicone-urethane copolymer was dissolved in 81.5g Dimethylformamide/Tetrahydrofuran (DMF/THF) for 24 hours at room temperature. 4g of CHA was added into the polymer solution and stirred for another 24 hours at room temperature. Table 1 shows the amounts ofpolymers, CHA, solvent mixture of the coating solutions. 16 French (fr) full assembly Foley catheters were spray coated with each solution with a CHA target loading of 650 µg/cm. The coated catheters were dried in 70°C oven for 48 hours to remove solvents.

### Example 2: Coating with 7.25% silicone-urethane copolymer / 4% CHA solution

14.5g of silicone-urethane copolymer was dissolved in 162g DMF/THF for 24 hours at room temperature. 4g of CHA was added into the polymer solution and stirred for another 24hours at room temperature. Table 1 shows the amounts of polymers, CHA, solvent mixture of the coating solutions.16fr full assembly Foley catheters were spray coated with each solution with a CHA target loading of 650 µg/cm. The coated catheters were dried in 70°C oven for 48 hours to remove solvents.

### Example 3: Impregnate 16fr Foley catheter body in 10% CHA in 90%/10% v/v THF/Methanol

4-in segments of catheter body were cut and placed in 15ml centrifuge tubes. Silicone segments were impregnated with CHA solution for 1hour and air dried for another hour. Segments were then vacuum dried at 35°C overnight, dip rinsed in de-ionized (DI) water for 10 seconds and vacuum dried overnight again. Silicone-urethane percentages obtained are shown in Table 1 below:

**Table 1: Silicone-urethane loaded CHA coating solutions**

| Polymer Soln ID | Silicone-urethane 67.5%Si/32.5 PU | Silicone-urethane 70% Si/30 PU | Silicone-urethane 20%Si/80% PU | CHA (g) | DMF/THF (g) |
|---|---|---|---|---|---|
| 1 | 14.502 | | | 4.003 | 81.5 |
| 2 | | 14.501 | | 4.008 | 82 |
| 3 | | | | 4.07 | 162 |
| 4 | | | 14.507 | 4.01 | 162 |
| 5 (Control) | 14.5 | | | | |

### Example 4: Determination of Water content of Silicone-urethane copolymers

Three different silicone-urethane copolymer grades were used in water absorption determination. The resins were vacuumed dried at 70°C for 24 hours prior to testing. The vacuum dried resins were subjected to steam for one (1) hour and allowed to air dry for one (1) hour. The water uptake by the resins was analyzed by loss-in-weight method using the Computrac Vapor Pro (Arizona Instrument of Chandler AZ 85225 USA). Table 2 below shows the water uptake by the three resins.

**Table 2: Water uptake by Silicone-urethane copolymer**

| Polymer type | Si-urethane ratio | % moisture |
|---|---|---|
| Si | 100:0 | 0 |
| Si-Urethane copolymer | 67.5:32.5 | 0.74 |
| Si-Urethane copolymer | 70:30 | 0.93 |
| Si-Urethane copolymer | 20:80 | 1.78 |

### Example 5: Elution of chlorhexidine from coatings into synthetic urinary medium

Three 1 cm segments were placed into individual wells of 48 well storage plates. 1 mL of synthetic urine was added to each of the wells along with 1 blank well as a control. Plates were sealed with adhesive and incubated in a 37°C oven. After 1 day The segments were transferred to new wells to which 1 mL of fresh media was added. Additional transfers took place on day 3 and day 7. Media from each of the original wells was analyzed for CHA as describe below.

The synthetic urine media was first prepared for injection onto a high-petfoffnmcc liquid chromatography (HPLC) analyzer. 100 µl of sample was removed from each well and placed into a 1.7mL tube (Eppendorf of Hamburg Germany). 400 µl of 75:25 acrylonitrile (ACN):0.2% Trifluoroacetic acid (TFA) in water was added to each tube. The tubes were gently shaken to mix, and then centrifuged at 10,000 RPM for 5 minutes. Media was then aliquoted into HPLC vials for analysis. The following tables demonstrate the static release kinetics ofCHA in synthetic urinary medium.

**Table 3: Cumulative Release of CHA from all coated samples**

| Day | Initial CHA (ug/cm) | 1 | 3 | 7 | 10 | 14 |
|---|---|---|---|---|---|---|
| Control (100% Si) | 591.0 | 8 | 16 | 24 | N/A | N/A |
| Si-Urethane copolymer (67.5 Si/32.5 PU) | 594.5 | 240 | 289 | 338 | 361 | 387 |
| Si-Urethane copolymer (70 Si/30PU) | 564.2 | 194 | 249 | 317 | 350 | 379 |
| Si-Urethane copolymer (20 Si/80 PU) | 604.3 | 344 | 480 | 545 | 570 | 580 |
| N/A: CHA ceased clouting | | | | | | |

**Table 4: Cumulative Percent Release of CHA from all coated samples**

| Day | Initial CHA (ug/cm) | 1 | 3 | 7 | 10 | 14 |
|---|---|---|---|---|---|---|
| Control (100% Si) | 591.0 | 1% | 3% | 4% | N/A | N/A |
| Si-Urethane copolymer (67.5 Si/32.5 PU) | 594.5 | 40% | 49% | 57% | 61% | 65% |
| Si-Urethane copolymer (70 Si/30PU) | 564.2 | 34% | 44% | S6% | 62% | 67% |
| Si-Urethane copolymer (20 Si/80 PU) | 604.3 | 579, | 80% | 90% | 94% | 96% |
| N/A: CHA ceased eluting | | | | | | |

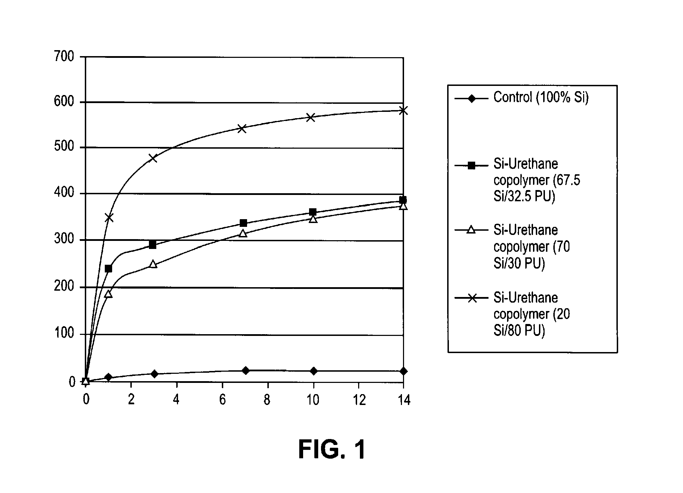

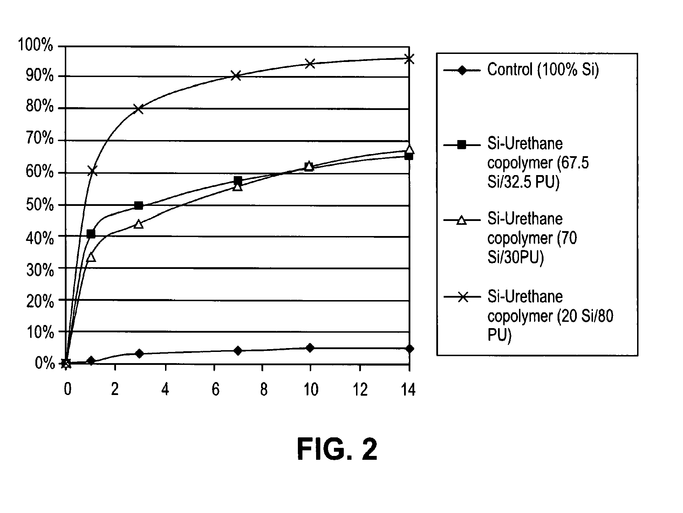

FIG. 1 is a graph of cumulative amount of chlorhexidine released versus day for different copolymer of silicone-urethane according to embodiments of the invention on suitable silicone urinary catheters. As shown in FIG. 1, CHA elutes from the 20 Si/80 Pu significantly more quickly than the 67.5 Si/32.5 Pu and 70 Si/30 Pu samples. This rapid release of the CHA may negatively impact adjacent tissues. In contrast, the 67.5 Si/32.5 Pu and 70 Si/30 Pu samples release CHA at a more gradual rate. FIG 2 is a graph of cumulative percent chlorhexidine release versus day for different copolymer of silicone-urethane according to embodiments of the invention on suitable silicone urinary catheters. As shown in FIG. 2, again the CHA clues from the 20 Si/80 Pu significantly more quickly than the 67.5 Si/32.5 Pu and 70 Si/30 Pu samples with nearly all of the CHA in the coating being released within 7 days. In contrast, only about 55% of the CHA has been released from the 67.5 Si/32.5 Pu and 70 Si/30 Pu samples by day 7 and only about 65% by day 14. This more controlled release facilitates sustained antimicrobial activity for greater than 2 weeks. All samples were tested in vitro for CHA release for a duration of 14 days and the results are shown in Tables 3 & 4 and FIGS. 1 & 2. The control sample yielded negligible CHA elution beyond day 7. Most of the CHA was released (80%-90%) by day 7 for the silicone-urethane coating with majority urethane (high water content) and was almost entirely depleted by day 14. In contrast the less hydrophilic samples with majority silicone in the copolymer released CHA through 14 days and significant additional CHA remained in the coating at day 14 that would be capable of extending release well beyond 14 days.

### Example 6: Zone of inhibition testing of chlorhexidine coatings with different Si-PU contents

Silicone-urethane copolymer was dissolved in DMF/THF mixture by stirring for 24 hours at room temperature. Then CHA was added into the polymer solution and stirred for another 24hours at room temperature. The Table below shows the Si/Urethane ratio, mass (grams) of polymer, CHA and solvent mixture used for coating. 16fr full assembly Foley catheters were spray coated with each solution with a CHA target loading of 400 µg/cm. The coated catheters were dried at 70°C for 48 hours to remove solvents.

**Table 5: Silicone-urethane loaded CHA coating solutions**

| Si-Urethane copolymer (70% Si/30 PU) | Si-urethane copolymer (20% Si /80%PU | CHA (g) | DMF/THF (g) |
|---|---|---|---|
| 7.31 | | 2.06 | 41 |
| | 7.25 | 2.10 | 90.1 |

For 100% Si controls, 4-in segments of Si Foley catheter bodies were also cut and placed in 15ml centrifuge tubes. The segments were soaked in 10% CHA solution (90/10 THF/Methanol) and then quick rinsed three times with methanol to remove free-drug. The treated segments were vacuum dried at 35oC overnight. CHA content was tested by exhaustive extraction in THF followed by HPLC analysis. Results are tabulated below:

**Table 6: Silicone loaded CHA controls**

| Sample ID | IntialCHA(ug/cm) |
|---|---|
| Coated with 20/80 SWrethane copolymer | 398 |
| Coated with 70/30 Si/Urethane copolymer | 442 |
| Control - 1000/o Si | 372 |

Zones of inhibition were measured by cutting catheter segments into 1.0cm size pieces. 150 x 1. 5mm Petri dishes were filled with Mueller Hintan II Agar then streaked with a Pseudomonas Aeruginosa (ATCC 27853) inoculum that was diluted to match a 1.0 McFarland standard (3 x 108 colony forming units/ml). Catheter segments were inserted vertically into the plate prior to streaking. Testing was performed in triplicate. The plates were allowed to incubate 24-48 hours at 37°C. Each plate contained an untreated control as well as the test articles. After incubation the zone of inhibition around each catheter segment was read as the distance (in mm) between the catheter surface and nearest approach of the Pseudomonal lawn. Following reading of the zone the catheter segments were transferred to a freshly inoculated plate and allowed to incubate further. Mean zone of inhibition (in mm) as a function of day is tabulated below:

**Table 7: Silicone loaded CHA controls**

| Coating Type | Day 1 | Day 2 | Day 4 | Day 5 | Day 7 | Day 8 |
|---|---|---|---|---|---|---|
| 100% Si | 1.5 | 0 | 0 | 0 | 0 | 0 |
| 70/30 Si/Uredme | 10.5 | 4.8 | 2.1 | 2.4 | 1.7 | 0.7 |
| 20/80 Si/Urethane | 9.9 | 3.7 | 2.7 | 0 | 0 | 0 |
| Untreated catheter | 0 | 0 | 0 | 0 | 0 | 0 |

The results show that the 70/30 Si/Urethane coating composition was able to sustain a zone of inhibition longer than the other Si compositions.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A medical device having an antimicrobial agent, the medical device comprising:
a base material; and
a chlorhexidine or a pharmaceutically acceptable salt thereof disposed in the base material sufficient to reduce microbial growth, the base material including a polymer having a silicone monomer and a urethane monomer.

2. The medical device according to claim 1, wherein the base material has a silicone:urethane ratio (wt/wt) of about 50:50 to about 80:20.

3. The medical device according to claim 2, wherein the base material has a silicone:urethane ratio (wt/wt) of about 65:35 to about 70:30.

4. The medical device according to claim 1, wherein the base material includes a carbonate monomer.

5. The medical device according to claim 1, wherein the carbonate monomer is present in the base material at about 10-40% (wt/wt) carbonate.

6. The medical device according to claim 1, further comprising:
a first layer including a core material; and
a second layer including the base material.

7. The medical device according to claim 6, further comprising:
a plurality of layers of the core material; and
a plurality of layers of the base material.

8. The medical device according to claim 1, further comprising:
a first region including a core material; and
a second region including the base material.

9. The medical device according to claim 7, further comprising:
a plurality of regions of the core material; and
a plurality of regions of the base material.

10. The medical device according to claim 1, wherein the chlorhexidine comprises:
a chlorhexidine diacetate.

11. The medical device according to claim 1, wherein the chlorhexidine comprises:
a chlorhexidine/fatty acid salt, wherein the chlorhexidine/fatty acid salt is a neutralization product of chlorhexidine base and a fatty acid having between 12 and 18 carbon atoms.

12. The medical device according to claim 1, wherein the chlorhexidine comprises:
a mixture of chlorhexidine base and a pharmaceutically acceptable salt thereof.

13. The medical device according to claim 1, further comprising:
a bioactive agent including one or more of an antibiotic, antiseptic, chemotherapeutic, antimicrobial peptide, mimetic, antithrombogenic, fibrinolytic, anticoagulant, anti-inflammatory, anti-pain, antinausea, vasodilator, antiproliferative, antifibrotic, growth factor, cytokine, antibody, peptide and peptide mimetics, and nucleic acid.

14. A medical catheter according to any one of claims 1-13, further comprising:
an elongated hollow tube having an exterior surface including the base material.
